# EUROPEAN PATENT APPLICATION

(11) **EP 1 319 710 A1**
(43) Date of publication of application: **18.06.2003**
(21) Application number: 01965691.7
(22) Date of filing: 18.09.2001
(51) Int. Cl.: C12N 15/09, C12Q 1/68

(54) **METHOD OF FORMING COMPLEX**

(30) Priority: 19.09.2000 JP 2000284420; 09.05.2001 JP 2001139286; 12.06.2001 JP 2001177537
(71) Applicant: TAKARA BIO INC., Otsu-shi, Shiga 520-2193 (JP)
(72) Inventor: SAGAWA, Hiroaki, Kusatsu-shi, Shiga 525-0025 (JP); ENOKI, Tatsuji, Otsu-shi, Shiga 520-0865 (JP); KOBAYASHI, Eiji, Otsu-shi, Shiga 520-2153 (JP); TOMONO, Jun, Kusatsu-shi, Shiga 525-0025 (JP); KATO, Ikunoshin, Uji-shi, Kyoto 611-0028 (JP)
(74) Representative: Grund, Martin, Dr.
(86) International application number: JP0108089
(87) International publication number: WO02024901

(57) **Abstract**

A method of forming a complex consisting of a double-stranded nucleic acid and oligonucleotide(s) characterized by comprising: the step of mixing a double-stranded nucleic acid with at least one oligonucleotide to give a reaction mixture, wherein the oligonucleotide is a chimeric oligonucleotide containing at least a member selected from among deoxyribonucleotides and nucleotide analogs and a ribonucleotide and having a sequence substantially complementary to the base sequence of one of the strands of the double-stranded nucleic acid as described above; and the step of incubating the reaction mixture to form a complex under such conditions that the above-described double-stranded nucleic acid is not denatured.

## Description

### Technical Field

The present invention relates to a method for forming a complex consisting of a double-stranded nucleic acid and an oligonucleotide, a method for creating a replication origin on a nucleic acid, and a method for replicating a nucleic acid, which are useful in a field of genetic engineering.

### Background Art

Progresses in studies in a field of molecular biology have brought various research techniques. Among these, nucleic acid hybridization techniques are quite useful techniques that allow detection and quantification of nucleic acids of interest, and have been utilized in various fields in addition to the field of molecular biology in manners suitable for the respective objects.

Basically, hybridization is a reaction of base pairing of single-stranded nucleic acids complementary to each other. Thus, it is necessary to first separate (denature) a double-stranded nucleic acid into two single strands for hybridization between a double-stranded nucleic acid and a single-stranded nucleic acid, or double-stranded nucleic acids. Heating or alkali treatment is used for the denaturation. However, it is impossible to carry out such treatment in the presence of a protein or the like without abolishing the activity of the protein.

Furthermore, in the course of hybridization using a double-stranded nucleic acid, formation of the original double-stranded nucleic acid at some frequency is inevitable.

It is known that a triple helix can be formed between an undenatured double-stranded nucleic acid and a single-stranded nucleic acid (Nucleic Acids Research, 16:11431-11440 (1988)). However, application of the formation of such a triple helix is limited to a region of a double-stranded nucleic acid formed by special sequences, i.e., a sequence of high purine content and a sequence of high pyrimidine content.

It is known that a complex of a double-stranded nucleic acid and a single-stranded nucleic acid is formed under nondenaturing conditions in the presence of a RecA protein involved in homologous recombination. However, this phenomenon is based on the activity of the RecA protein, and no technique for nonenzymatically forming a similar complex is known.

A technique for maintaining or amplifying an isolated nucleic acid molecule is indispensable to studies in a field of genetic engineering. The maintenance or amplification is usually accomplished by inserting the isolated nucleic acid into an appropriate vector to make a recombinant DNA molecule. Such a recombinant DNA molecule can be stably maintained being isolated or harbored in an appropriate-host. -Furthermore, it is possible to increase the number of molecules by culturing the host harboring the recombinant DNA molecule to increase the cell number, if necessary.

A number of vectors derived from plasmids, phages, viruses and the like are known. In addition, vectors artificially modified to have various functions suitable for their uses have been developed and are commercially available.

Utilization of a host is indispensable for maintaining or amplifying a gene using a vector. Accordingly, it is difficult to construct a recombinant DNA molecule in which a nucleic acid molecule of interest is incorporated into a vector, for example, if the nucleic acid molecule of interest is harmful to the host (e.g., if the vector is to contain a gene encoding a product lethal to the host).

Amplification of a nucleic acid molecule of interest without the mediation of a host by replicating a recombinant DNA molecule outside a host cell (i.e., in vitro) may be considered. However, since every known vector is replicated from a defined replication origin by an inherent mechanism, no general-purpose means of artificially replicating a nucleic acid molecule is known.

### Objects of Invention

The main object of the present invention is to provide a method for forming a complex consisting of a double-stranded nucleic acid and a single-stranded nucleic acid without denaturation, and a convenient general-purpose method for replicating a nucleic acid molecule without the use of a host organism.

### Summary of Invention

As a result of intensive studies, the present inventors have found that a chimeric oligonucleotide containing a ribonucleotide is annealed to an undenatured double-stranded nucleic acid to form a complex in vitro, the complex functions as a replication origin on the nucleic acid, and the nucleic acid can be replicated from the replication origin. Thus, the present invention has been completed.

The first aspect of the present invention relates to a method for forming a complex consisting of a double-stranded nucleic acid and an oligonucleotide, the method comprising:
(a) mixing a double-stranded nucleic acid and at least one oligonucleotide to prepare a reaction mixture, wherein the oligonucleotide is a chimeric oligonucleotide containing at least a ribonucleotide as well as a member selected from a group consisting of a deoxyribonucleotide and a nucleotide analog, and has a sequence substantially complementary to the nucleotide sequence of one of the two strands of the double-stranded nucleic acid; and
(b) incubating the reaction mixture to form a complex under conditions under which the double-stranded nucleic acid is not denatured.

Examples of the double-stranded nucleic acids used in the first aspect include a linear DNA, a circular DNA and a genomic DNA.

The oligonucleotide may function as a primer for synthesizing a DNA complementary to one of the two strands of the double-stranded nucleic acid, and/or it may be labeled.

The second aspect of the present invention relates to a method for detecting a double-stranded nucleic acid having a target nucleotide sequence, the method comprising:
(a) forming a complex consisting of a double-stranded nucleic acid and an oligonucleotide according to the method of the first aspect, wherein the oligonucleotide is a chimeric oligonucleotide containing at least a ribonucleotide as well as a member selected from a group consisting of a deoxyribonucleotide and a nucleotide analog, and has a sequence substantially complementary to a target nucleotide sequence; and
(b) detecting the oligonucleotide forming the complex.

The third aspect of the present invention relates to a method for creating a replication origin on a double-stranded nucleic acid, the method comprising:
(a) mixing a double-stranded nucleic acid and at least one oligonucleotide to prepare a reaction mixture, wherein the oligonucleotide is a chimeric oligonucleotide containing at least a ribonucleotide as well as a member selected from a group consisting of a deoxyribonucleotide and a nucleotide analog, allows extension from its 3' terminus by a DNA polymerase, and has a sequence substantially complementary to the nucleotide sequence of one of the two strands of the double-stranded nucleic acid; and
(b) incubating the reaction mixture to form a complex under conditions under which the double-stranded nucleic acid is not denatured.

Examples of the double-stranded nucleic acids used in the third aspect include a linear DNA, a circular DNA and a genomic DNA.

The fourth aspect of the present invention relates to a method for replicating a nucleic acid, the method comprising:
synthesizing a DNA complementary to one of the two strands of a double-stranded nucleic acid from a replication origin created according to the method of the third aspect in the presence of a DNA polymerase.

The DNA polymerase used in the fourth aspect is exemplified by an enzyme that has a strand displacement activity.

### Brief Description of Drawings

Figure 1: a figure illustrating the results of agarose gel electrophoresis of DNA fragments amplified from replication origins created according to the method of the present invention.
Figure 2: a figure illustrating the results of agarose gel electrophoresis of DNA fragments amplified from replication origins created according to the method of the present invention.
Figure 3: a figure illustrating the results of agarose gel electrophoresis of DNA fragments amplified from replication origins created according to the method of the present invention.
Figure 4: a figure illustrating the results of agarose gel electrophoresis of DNA fragments amplified from replication origins created according to the method of the present invention.

### Detailed Description of the Invention

As used herein, a complex consisting of a double-stranded nucleic acid and an oligonucleotide refers to a complex in which a double-stranded nucleic acid and an oligonucleotide are non-covalently bound each other. There is no specific limitation concerning the form thereof. For example, one forming a triple helix and one in which an oligonucleotide is base-pairing only with one of the two strands of a double-stranded nucleic acid are included. A complex consisting of a double-stranded nucleic acid and an oligonucleotide may be merely referred to as a "complex" hereinafter.

According to the present invention, a nucleotide is usually a deoxyribonucleotide or a ribonucleotide. Optionally, an analog or a derivative (modification) of a nucleotide may be included.

As used herein, a deoxyribonucleotide refers to a nucleotide of which the sugar portion is composed of D-2-deoxyribose. The deoxyribonucleotides include, for example, ones having adenine, cytosine, guanine or thymine as the base portion. Furthermore, the deoxyribonucleotides also include a deoxyribonucleotide analog having a modified base such as 7-deazaguanosine or inosine as the base portion.

As used herein, a ribonucleotide refers to a nucleotide of which the sugar portion is composed of D-ribose. The ribonucleotides include ones having adenine, cytosine, guanine or uracil as the base portion. The ribonucleotides also include ribonucleotide analogs and modified ribonucleotides such as a modified ribonucleotide in which the oxygen atom of the phosphate group at the α-position is replaced by a sulfur atom (an (α-S) ribonucleotide).

As used herein, a replication origin refers to a site that can functions as a reaction initiation point for replication of a double-stranded nucleic acid, i.e., synthesis of a DNA having the nucleotide sequence of the nucleic acid. It is not limited to a site characterized by a certain nucleotide sequence.

Hereinafter, the present invention will be described in detail.

### (1) Chimeric oligonucleotide used according to the present invention

The chimeric oligonucleotide used in the method of the present invention is a chimeric oligonucleotide that contains at least a ribonucleotide as well as a member selected from a group consisting of a deoxyribonucleotide and a nucleotide analog. The chimeric oligonucleotides include an oligonucleotide that contains at least one selected from the group consisting of a modified ribonucleotide, a modified deoxyribonucleotide, a ribonucleotide analog and a deoxyribonucleotide analog.

For example, a nucleotide analog having a base such as inosine or 7-deazaguanine as the base portion, or nucleotide analog having a ribose derivative such as a locked nucleic acid (LNA; Proc. Natl. Acad. Sci. USA, 97:5633-5638 (2000); WO 99/14226) can be used as a nucleotide analog used according to the present invention. The modified ribonucleotide is exemplified by an (α-S) nucleotide in which the oxygen atom attached to the phosphate group is replaced by a sulfur atom, or a nucleotide to which a labeling compound is added. Furthermore, the chimeric oligonucleotide according to the present invention may contain a peptide nucleic acid (PNA; Nature, 365:566-568 (1993)).

There is no specific limitation concerning the chimeric oligonucleotide used according to the present invention as long as it can form a complex with a double-stranded nucleic acid of interest. Also, there is no specific limitation concerning the position at which a ribonucleotide is contained. For example, one in which a ribonucleotide is positioned at the 3' terminus or on the 3'-terminal side of the primer can be used. Furthermore, the number of the ribonucleotides is not specifically limited. A chimeric oligonucleotide in which half or less of the constituting bases are ribonucleotides is exemplified.

As used herein, 3'-terminal side refers to a portion from the center to the 3' terminus of a nucleic acid such as an oligonucleotide. Likewise, 5' terminal side refers to a portion from the center to the 5' terminus of a nucleic acid.

The chimeric oligonucleotide used in the method of the present invention has a nucleotide sequence substantially complementary to a portion of the nucleotide sequence of the double-stranded nucleic acid with which the chimeric oligonucleotide is to form a complex. As used herein, "a substantially complementary nucleotide sequence" means a nucleotide sequence that can anneal to one of the two strands of the double-stranded nucleic acid under conditions under which a nucleic acid can assume a double-stranded form, for example, under the conditions under which the present invention is carried out.

Although the length of the chimeric oligonucleotide used in the method of the present invention is not specifically limited, for example, it is from 12 nucleotides to 100 nucleotides, preferably from 15 nucleotides to 40 nucleotides.

Incorporation of a nucleotide analog into a chimeric oligonucleotide is effective for suppressing the formation of high-order structure of the chimeric oligonucleotide itself. Incorporation of a nucleotide analog that can form a strong base-pairing bond such as an LNA is useful for highly efficient formation of a complex.

Such a chimeric oligonucleotide can be synthesized to have a desired nucleotide sequence, for example, using the 394 type DNA synthesizer from Applied Biosystems Inc. (ABI) according to a phosphoramidite method. Alternatively, any methods including a phosphate triester method, an H-phosphonate method and a thiophosphonate method may be used to synthesize the chimeric oligonucleotide.

### (2) Method for forming complex of the present invention

The present invention provides a method for forming a complex composed of the chimeric oligonucleotide as described in (1) above and a double-stranded nucleic acid having a nucleotide sequence substantially complementary to the chimeric oligonucleotide.

There is no specific limitation concerning the double-stranded nucleic acid used in the method of the present invention. The method can be applied to linear and circular ones. For example, one can create a replication origin on a plasmid DNA, a genomic DNA, a fragment thereof, a DNA fragment amplified by PCR or the like. The method of the present invention can be applied to either a naturally occurring nucleic acid or an artificially prepared nucleic acid.

According to the present invention, it is possible to form a complex in vitro only by mixing the chimeric oligonucleotide as described in (1) above and a double-stranded nucleic acid under appropriate conditions without denaturing the double-stranded nucleic acid into single strands. Although there is no specific limitation concerning the conditions, conditions under which a double-stranded nucleic acid to be used is not denatured into single strands are usually used. For example, a complex can be formed at a temperature of 0 to 70°C at pH of 6.0 to 9.5, preferably 7.0 to 9.2.

A reaction mixture for forming a complex may contain a buffering component for keeping appropriate pH, a neutral salt for adjusting ionic strength or other components. Furthermore, it may contain an enzyme to be allowed to act on the formed complex and various components required for exhibiting the activity of the enzyme. Furthermore, it is expected that addition of dimethyl sulfoxide (DMSO), glycerol, polyethylene glycol and/or formamide reduces nonspecific annealing of a chimeric oligonucleotide.

Although there is no specific limitation concerning the amount of the chimeric oligonucleotide used, for example, the amount in a reaction volume of 50 µl may range from 1 to 1000 pmol, preferably from 10 to 150 pmol.

One can synthesize a DNA strand having a sequence complementary to one of the two strands of a double-stranded nucleic acid by using the complex formed according to the method of the present invention to synthesize a DNA from the 3' terminus of a chimeric oligonucleotide. In this case, it is possible to form a complex in a reaction mixture containing a DNA polymerase, deoxynucleotide triphosphates as substrates, a magnesium salt and the like for conducting DNA synthesis at the same time.

Although it is not intended to limit the present invention; the method for forming a complex of the present invention can be carried out in the presence of a ribonuclease H (RNase H). Both mesophilic ribonuclease H and heat-resistant ribonuclease H can be preferably used according to the present invention. For example, in addition to an RNase H from E. coli, a commercially available Hybridase™ Thermostable RNase H (Epicenter Technologies) as well as an RNase from a thermophilic bacterium of genus Bacillus, a bacterium of genus Thermus, a bacterium of genus Pyrococcus, a bacterium of genus Thermotoga or the like can be used. Furthermore, both naturally occurring ribonucleases and variants can be preferably used. A heat-resistant RNase H from Pyrococcus furiosus can be preferably used according to the present invention. This RNase H can be obtained from Pyrococcus furiosus DSM3638. Alternatively, it can be obtained utilizing a gene encoding the enzyme as described in Referential Example below.

If the present invention is carried out in the presence of a ribonuclease H, it is preferable to carry out it in a reaction mixture in which the ribonuclease H to be used can exhibit its activity.

Although it depends on the type of RNase H to be used, a reaction mixture containing an appropriate buffering component and a magnesium salt and/or a manganese salt is usually used. Although it is not intended to limit the present invention, for example, magnesium chloride, magnesium acetate, magnesium sulfate, manganese chloride, manganese acetate or manganese sulfate can be preferably used as a magnesium salt/a manganese salt at a final concentration of 1 mM to 20 mM, preferably 2 mM to 10 mM. A reaction mixture containing, for example, Bicine, Tricine, HEPES, Tris and a phosphate (such as sodium phosphate and potassium phosphate) as a buffering component can be used. A reaction mixture containing the buffering component at a final concentration of 5 mM to 100 mM, preferably 10 mM to 50 mM at pH of 6.0 to 9.5, preferably 7.0 to 9.2 is used, without limitation.

The present invention is not limited only to formation of a complex, but encompasses various operations comprising a step of forming the above-mentioned complex without denaturation.

The method for forming a complex of the present invention can be utilized to detect a nucleic acid as described below. In addition, by forming a complex in a region of a nucleic acid that has a specific function (e.g., a promoter region), it is possible to suppress or inhibit the function.

### (3) Method for detecting nucleic acid of the present invention

Based on the above-mentioned method for forming a complex, the present invention provides a method for detecting a double-stranded nucleic acid having a target nucleotide sequence.

The chimeric oligonucleotide as described in (1) above forms a complex with a double-stranded nucleic acid having a nucleotide sequence substantially complementary to the chimeric oligonucleotide. Thus, a double-stranded nucleic acid having a nucleotide sequence substantially complementary to the chimeric oligonucleotide can be detected by examining the formation of the complex.

In this embodiment, it is preferable to use a labeled chimeric oligonucleotide. There is no specific limitation concerning the type of the label. For example, radioisotopes (³²P, etc.), dyes, fluorescent substances, luminescent substances, various ligands (biotin, digoxigenin, etc.), enzymes and the like can be used. The existence of the labeled chimeric oligonucleotide can be confirmed by a detection method suitable for the label. In case of a ligand that cannot be detected directly, it may be used in combination with a substance that is capable of binding to the ligand and labeled with a detectable label. For example, a target nucleic acid can be detected with high sensitivity by using a chimeric oligonucleotide labeled with a ligand and an anti-ligand antibody labeled with an enzyme in combination, and amplifying the signal.

Furthermore, the method for detecting a target nucleic acid of the present invention can be carried out in the presence of a ribonuclease H as described in (2) above.

### (4) Method for creating replication origin of the present invention

The method for creating a replication origin of the present invention comprises:
(a) mixing a double-stranded nucleic acid and at least one oligonucleotide to prepare a reaction mixture, wherein the oligonucleotide is a chimeric oligonucleotide containing at least a ribonucleotide as well as a member selected from a group consisting of a deoxyribonucleotide and a nucleotide analog, allows extension from its 3' terminus by a DNA polymerase, and has a sequence substantially complementary to the nucleotide sequence of one of the two strands of the double-stranded nucleic acid; and
(b) incubating the reaction mixture to anneal the oligonucleotide to the double-stranded nucleic acid under conditions under which the double-stranded nucleic acid is hot denatured.

There is no specific limitation concerning the double-stranded nucleic acid used in the method for creating a replication origin of the present invention. Any one of those described in (2) above may be used. Also, the reaction conditions used for the method for forming a complex of the present invention as described in (2) above can be used.

The chimeric oligonucleotide that allows extension from its 3' terminus by a DNA polymerase as described in (1) above can serve as a primer for DNA synthesis by a DNA polymerase when it forms a complex with a double-stranded nucleic acid. The DNA polymerase synthesizes a DNA complementary to one of the two strands of the double-stranded nucleic acid from the 3' terminus of the chimeric oligonucleotide to replicate the DNA. Thus, the complex functions as a replication origin on the double-stranded nucleic acid.

According to the present invention, it is possible to create a replication origin at any position on a double-stranded nucleic acid depending on the nucleotide sequence of the chimeric oligonucleotide. The number of the replication origins to be created is not limited to one. Plurality of replication origins can be created at the same time using plurality of chimeric oligonucleotide primers.

Furthermore, the method for creating a replication origin of the present invention can be carried out in the presence of a ribonuclease H as described in (2) above.

### (5) Method for replicating nucleic acid of the present invention

The method for replicating a nucleic acid of the present invention is accomplished by synthesizing a DNA strand complementary to one of the two strands of a double-stranded nucleic acid from the 3' terminus of a chimeric oligonucleotide contained in a replication origin created on the double-stranded nucleic acid according to the method as described in (4) above by the action of a DNA polymerase.

The DNA synthesis is initiated utilizing the chimeric oligonucleotide as a primer, and proceeds utilizing as a template, among the two strands of the double-stranded nucleic acid, a strand having a nucleotide sequence substantially complementary to the chimeric oligonucleotide.

The DNA polymerase used in the method for replicating a nucleic acid of the present invention is not limited to specific one as long as it has an activity of synthesizing a new DNA strand using a DNA strand as a template. For example, Pol I-type DNA polymerases (Escherichia coli DNA polymerase I, Klenow fragment, Taq DNA polymerase, etc.), α-type DNA polymerases [a DNA polymerase from Pyrococcus furiosus (Stratagene), VENT DNA polymerase (New England Biolabs), KOD DNA polymerase (Toyobo), DEEP VENT DNA polymerase (New England Biolabs)] and non-α, non-Pol I-type DNA polymerases (the DNA polymerase as described in WO 97/24444) can be used according to the present invention. Furthermore, plurality of DNA polymerases may be mixed and used. It is preferable to use a DNA polymerase lacking its 5'→3' exonuclease activity for replicating a linear double-stranded nucleic acid.

A DNA polymerase having a strand displacement activity on a DNA can be used according to the present invention. "A strand displacement activity" refers to an activity that can effect a strand displacement, that is, that can proceed DNA replication on the basis of the sequence of the nucleic acid as the template while displacing the DNA strand to release the complementary strand that has been annealed to the template strand. In addition, a DNA strand released from a nucleic acid sequence as a template as a result of a strand displacement is referred to as "a displaced strand" herein.

In one preferred embodiment, the method for replicating a nucleic acid of the present invention is carried out under high temperature conditions (e.g., 45 to 70°C). In this embodiment, heat-resistant DNA polymerases, preferably DNA polymerases derived from thermophilic bacteria of genus Bacillus such as Bacillus caldotenax or Bacillus stearothermophilus as well as mutants of the DNA polymerases lacking their 5'→3' exonuclease activities are used. These DNA polymerases have the above-mentioned strand displacement activities.

A replication reaction can be carried out in a reaction mixture containing four dNTPs (dATP, dCTP, dGTP, dTTP) as substrates for a DNA polymerase and other components required for exhibiting the activity of the enzyme under reaction conditions suitable for the enzyme to be used. Naturally, the compositions and concentrations of the respective components in the reaction mixture, the pH of the reaction mixture, the reaction temperature, the reaction time and the like can be appropriately adjusted depending on the enzyme to be used or the nucleic acid to be replicated.

The step of replication reaction may be conducted subsequent to the step of creating a replication origin. Alternatively, these steps may be conducted at the same time. In this case, the DNA polymerase and other enzyme(s) to be used are selected and the reaction conditions are determined such that they exhibit their activities sufficiently.

The step of replication reaction can be conducted successively by using a nucleic acid amplification method in which a DNA polymerase having a strand displacement activity and a ribonuclease H are used in combination. Such a method is described in WO 00/56877 and can be conducted using a DNA polymerase having a strand displacement activity and a ribonuclease H as described above. In this case, it is preferable to make a chimeric oligonucleotide in a form suitable for this method.

In addition, it is possible to amplify a nucleic acid fragment from a region put between two replication origins positioned to face each other by replicating a nucleic acid from the replication origins at the same time. The present invention encompasses such an embodiment.

### Examples

The following examples further illustrate the present invention in detail but are not to be construed to limit the scope thereof.

### Referential Example

### Cloning of Pyrococcus furiosus RNase HII gene

### (1) Preparation of genomic DNA from Pyrococcus furiosus

2 L of a medium containing 1% Tryptone (Difco Laboratories), 0.5% yeast extract (Difco Laboratories), 1% soluble starch (Nacalai Tesque), 3.5% Jamarine S Solid (Jamarine Laboratory), 0.5% Jamarine S Liquid (Jamarine Laboratory), 0.003% MgSO₄, 0.001% NaCl, 0.0001% FeSO₄·7H₂O, 0.0001% CoSO₄, 0.0001% CaCl₂·7H₂O, 0.0001% ZnSO₄, 0.1 ppm CuSO₄ · 5H₂O, 0.1 ppm KAl(SO₄)₂, 0.1 ppm H₃BO₄, 0.1 ppm Na₂MoO₄ · 2H₂O and 0.25 ppm NiCl₂ · 6H₂O was placed in a 2-L medium bottle, sterilized at 120°C for 20 minutes, bubbled with nitrogen gas to remove dissolved oxygen, then Pyrococcus furiosus (purchased from Deutsche Sammlung von Mikroorganismen; DSM3638) was inoculated into the medium and cultured at 95°C for 16 hours without shaking. After cultivation, cells were collected by centrifugation.

The resulting cells were then suspended in 4 ml of 25% sucrose, 50 mM tris-HCl buffer (pH 8.0). 0.4 ml of a 10 mg/ml solution of lysozyme chloride (Nacalai Tesque) in water was added thereto. The mixture was reacted at 20°C for 1 hour. After reaction, 24 ml of a mixture containing 150 mM NaCl, 1 mM EDTA and 20 mM tris-HCl buffer (pH 8.0), 0.2 ml of 20 mg/ml proteinase K (Takara Shuzo) and 2 ml of 10% aqueous solution of sodium lauryl sulfate were added to the reaction mixture. The mixture was incubated at 37°C for 1 hour.

After reaction, the mixture was subjected to phenol-chloroform extraction followed by ethanol precipitation to prepare about 1 mg of genomic DNA.

### (2) Cloning of RNase HII gene

The entire genomic sequence of Pyrococcus horikoshii was published [DNA Research, 5:55-76 (1998)]. The existence of one gene encoding a homologue of RNase HII (PH1650) in the genome was known (SEQ ID NO:1, the home page of National Institute of Technology and Evaluation: http://www.nite.go.jp/).

Homology between the PH1650 gene (SEQ ID NO:1) and the partially published genomic sequence of Pyrococcus furiosus (the home page of University of Utah, Utah Genome Center: http://www.genome.utah.edu/sequence.html) was searched. As a result, a highly homologous sequence was found.

Primers 1650Nde (SEQ ID NO:2) and 1650Bam (SEQ ID NO:3) were synthesized on the basis of the homologous sequence.

A PCR was carried out in a volume of 100 µl using 200 ng of the Pyrococcus furiosus genomic DNA obtained in Referential Example (1) as a template, and 20 pmol of 1650Nde and 20 pmol of 1650Bam as primers. TaKaRa Ex Taq (Takara Shuzo) was used as a DNA polymerase for the PCR according to the attached protocol. The PCR was carried out as follows: 30 cycles of 94°C for 30 seconds, 55°C for 30 seconds and 72°C for 1 minute. An amplified DNA fragment of about 0.7 kb was digested with NdeI and BamHI (both from Takara Shuzo). The resulting DNA fragment was incorporated between the NdeI and BamHI sites in a plasmid vector pET3a (Novagen) to make a plasmid pPFU220.

### (3) Determination of nucleotide sequence of DNA fragment containing RNase HII gene

The nucleotide sequence of the DNA fragment inserted into pPFU220 obtained in Referential Example (2) was determined according to a dideoxy method.

Analysis of the determined nucleotide sequence revealed an open reading frame (ORF) presumably encoding RNase HII. The nucleotide sequence of the open reading frame is shown in SEQ ID NO:4. The amino acid sequence of RNase HII deduced from the nucleotide sequence is shown in SEQ ID NO:5.

Escherichia coli JM109 transformed with the plasmid pPFU220 is designated and indicated as Escherichia coli JM109/pPFU220, and deposited on September 5, 2000 (date of original deposit) at International Patent Organism Depositary, National Institute of Advanced Industrial Science and Technology, AIST Tsukuba Central 6, 1-1, Higashi 1-chome, Tsukuba-shi, Ibaraki 305-8566, Japan under accession number FERM BP-7654.

### (4) Preparation of purified RNase HII preparation

Escherichia coli HMS174(DE3) (Novagen) was transformed with pPFU220 obtained in Referential Example (2). The resulting Escherichia coli HMS174(DE3) harboring pPFU220 was inoculated into 2 L of LB medium containing 100 µg/ml of ampicillin and cultured with shaking at 37°C for 16 hours. After cultivation, cells collected by centrifugation were suspended in 66.0 ml of a sonication buffer [50 mM tris-HCl (pH 8.0), 1 mM EDTA, 2 mM phenylmethanesulfonyl fluoride (PMSF)] and sonicated. A supernatant obtained by centrifuging the sonicated suspension at 12000 rpm for 10 minutes was heated at 60°C for 15 minutes. It was then centrifuged at 12000 rpm for 10 minutes again to collect a supernatant. Thus, 61.5 ml of a heated supernatant was obtained.

The heated supernatant was subjected to RESOURSE Q column (Amersham Pharmacia Biotech) equilibrated with Buffer A [50 mM tris-HCl buffer (pH 8.0), 1 mM EDTA] and chromatographed using FPLC system (Amersham Pharmacia Biotech). As a result, RNase HII flowed through the RESOURSE Q column.

60.0 ml of the flow-through RNase HII fraction was subjected to RESOURSE S column (Amersham Pharmacia Biotech) equilibrated with Buffer A and eluted with a linear gradient of 0 to 500 mM NaCl using FPLC system. A fraction containing RNase HII eluted with about 150 mM NaCl was obtained.

2.0 ml of the RNase HII fraction was concentrated by ultrafiltration using Centricon-10 (Amicon). 250 µl of the concentrate was subjected to Superdex 200 gel filtration column (Amersham Pharmacia Biotech) equilibrated with 50 mM tris-HCl buffer (pH 8.0) containing 100 mM NaCl and 0.1 mM EDTA and eluted with the same buffer. As a result, RNase HII was eluted at a position corresponding to a molecular weight of 17 kilodalton. This molecular weight corresponds to that of the RNase HII in the monomeric form.

The thus eluted RNase HII was used as Pfu RNase HII preparation.

The RNase H activity of the thus obtained Pfu RNase HII preparation was measured as follows.

10 mM tris-HCl buffer (pH 8.0), 1 mM dithiothreitol (DTT, Nacalai Tesque), 0.003% bovine serum albumin (fraction V, Sigma), 4% glycerol, 20 µg/ml poly(dT) (Amersham Pharmacia Biotech) and 30 µg/ml poly(rA) (Amersham Pharmacia Biotech) were mixed together. The mixture was incubated at 37°C for 10 minutes and used as a substrate solution for measuring an RNase H activity.

1 µl of 1 M MnCl₂ was added to 100 µl of the substrate solution. The mixture was incubated at 40°C. An appropriate dilution of the Pfu RNase HII preparation was added to the mixture to initiate a reaction. After reacting at 40°C for 30 minutes, 10 µl of 0.5 M EDTA was added thereto to terminate the reaction. Absorbance at 260 nm was then measured.

As a result, the absorbance at 260 nm observed for the reaction mixture to which the Pfu RNase HII preparation was added was higher than that observed for the reaction mixture to which 10 µl of 0.5 M EDTA was added before the addition of the Pfu RNase HII preparation. Thus, it was shown that this preparation had an RNase H activity.

### Example 1

(1) RAW264.7 cells (ATCC TIB 71) were suspended in Dulbecco's modified Eagle's medium (Bio Whittaker, 12-604F) containing 10% fetal calf serum (Gibco) at a concentration of 1.5 x 10⁵ cells/ml. 5 ml of the suspension was added to each well of a 6-well microtiter plate and the plate was incubated at 37°C overnight in the' presence of 5% CO₂. 50 µl of 100 µg/ml solution of lipopolysaccharide (LPS, Sigma, L-2012) in water and 50 µl of 1000 U/µl solution of interferon-γ (IFN-γ, Genzyme Techne, 3485) in water were added to the well. The plate was incubated for additional 4 hours. An RNA was then prepared from the cells using RNeasy Mini Kit (Qiagen, 74104) according to the instructions attached to the kit.

A cDNA was prepared by incubating 60 µl of a mixture containing 3 µg of the thus prepared RNA, 10 mM tris-hydrochloride buffer (pH 8.3), 50 mM KCl, 5 mM MgCl₂, 1 mM each of dNTPs, 150 pmol of random 6mers primer, 60 U of ribonuclease inhibitor (Takara Shuzo, 2310A) and 15 U of Reverse Transcriptase XL (AMV) (Takara Shuzo, 2620A) at 30°C for 10 minutes, 42°C for 1 hour and then 99°C for 5 minutes for inactivating the enzyme using a thermal cycler (GeneAmp PCR System 9600, Applied Biosystems).

(2) Primers NS-PCR1 and NS-PCR2 having nucleotide sequences represented by SEQ ID NOS:6 and 7, respectively, were synthesized based on the nucleotide sequence of the mRNA for mouse inducible NO synthase (iNOS) (GeneBank accession no. NM-010927). A PCR was carried out using the pair of primers and the cDNA prepared in Example 1-(1) as a template to amplify a 741-bp DNA fragment. The fragment was purified using Suprec02 (Takara Shuzo) and then used for the following experiments.

(3) Chimeric oligonucleotide primers NS5 and NS6 represented by SEQ ID NOS:8 and 9, respectively, were synthesized based on the nucleotide sequence of the mRNA for mouse inducible NO synthase.

50 µl of a reaction mixture containing 1 µl of a solution containing the PCR-amplified DNA fragment obtained in Example 1-(2) at a concentration of 10 fg to 100 pg/µl or water (negative control), 50 pmol each of the primers NS5 and NS6, 0.5 mM each of dNTPs, 32 mM HEPES-potassium hydroxide buffer (pH 7.8), 100 mM potassium acetate, 4 mM magnesium acetate, 0.01% bovine serum albumin, 1% dimethyl sulfoxide, 0.0156 µg of Pfu RNase HII as described in Referential Example and 1 U of BcaBEST DNA polymerase (Takara Shuzo) was incubated at 60°C for 1 hour in a thermal cycler.

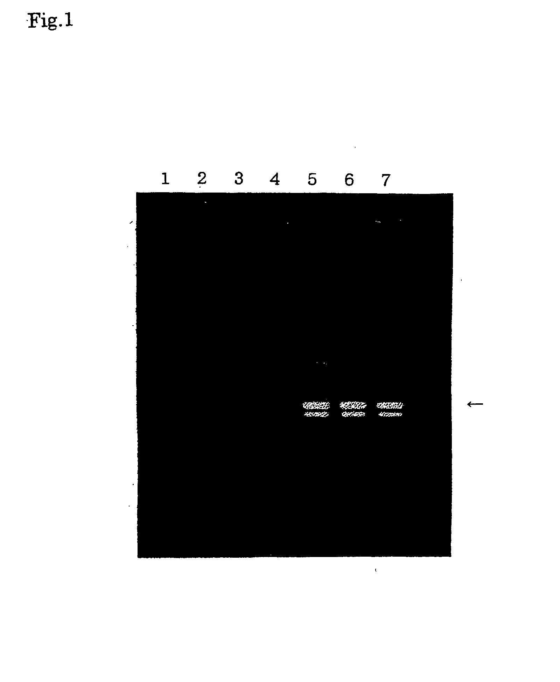

After reaction, 5 µl each of the reaction mixtures was analyzed by electrophoresis on 3.0% agarose gel. A photograph of the electrophoresis is shown in Figure 1.

Figure 1 is a photograph of the electrophoresis of the reaction products. Lane 1: 100 bp DNA ladder marker; lane 2: negative control (water); lane 3: 10 fg of the template; lane 4: 100 fg of the template; lane 5: 1 pg of the template; lane 6: 10 pg of the template; and lane 7: 100 pg of the template.

As shown in Figure 1, it was shown that a DNA fragment corresponding to the region of the template between the chimeric oligonucleotide primers was amplified when 1 pg or more of the template was used in the reaction. These results show that replication origins were formed as a result of annealing of the chimeric oligonucleotide primers to the template DNA although the template DNA was not denatured, and that the DNA polymerase effected DNA synthesis from the replication origins to amplify the DNA fragment between the replication origins.

### Example 2

(1) Chimeric oligonucleotide primers pDON-AI-1 and pDON-AI-2 each having three RNA residues at the 3' terminus were synthesized based the nucleotide sequence of the packaging region in a plasmid pDON-AI DNA (Takara Shuzo). The nucleotide sequences of these primers are shown in SEQ ID NOS:10 and 11.

(2) 50 µl of a reaction mixture containing 1 µl of a solution containing pDON-AI DNA (circular) at a concentration of 10 fg to 1 ng/µl or water (negative control), 50 pmol each of the primers pDON-AI-1 and pDON-AI-2, 0.5 mM each of dNTPs, 32 mM HEPES-potassium hydroxide buffer (pH 7.8), 100 mM potassium acetate, 4 mM magnesium acetate, 0.01% bovine serum albumin, 1% dimethyl sulfoxide, 0.0156 µg of Pfu RNase H as described in Referential Example and 1 U of BcaBEST DNA polymerase was incubated at 60°C for 1 hour in a thermal cycler.

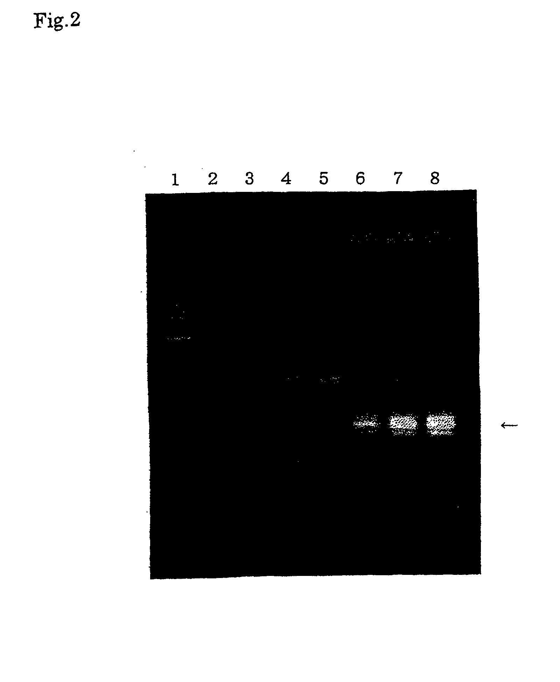

5 µl each of the reaction mixtures was analyzed by electrophoresis on 3.0% agarose gel. The results are shown in Figure 2.

Figure 2 is a photograph of the electrophoresis of the reaction products. Lane 1: 100 bp DNA ladder marker; lane 2: negative control (water); lane 3: 10 fg of the template; lane 4: 100 fg of the template; lane 5: 1 pg of the template; lane 6: 10 pg of the template; lane 7: 100 pg of the template; and lane 8: 1 ng of the template.

As shown in Figure 2, a DNA fragment corresponding to the region between the chimeric oligonucleotides used was amplified when 10 fg or more of the template was used. Thus, it was shown that a replication origin is created on a circular DNA molecule using an RNase H and a chimeric oligonucleotide primer.

### Example 3

(1) Chimeric oligonucleotide primers SEA-1 and SEA-2 each having three RNA residues at the 3' terminus were synthesized based on the nucleotide sequence of the enterotoxin A gene region of Staphylococcus aureus. The primer SEA-1 is a sense primer, whereas the primer SEA-2 is an antisense primer. The nucleotide sequences of the primers SEA-1 and SEA-2 are shown in SEQ ID NOS:12 and 13, respectively.

(2) 50 µl of a reaction mixture containing 1 µl of a solution containing 0.115 ng or 1.15 ng of a genomic DNA from Staphylococcus aureus (ATCC 13565) or 1 µl of water for a negative control, 50 pmol each of the primers SEA-1 and SEA-2, 0.5 mM each of dNTPs, 32 mM HEPES-potassium hydroxide buffer (pH 7.8), 100 mM potassium acetate, 4 mM magnesium acetate, 0.01% bovine serum albumin, 1% dimethyl sulfoxide, 0.0156 µg of Pfu RNase H as described in Referential Example and 1 U of BcaBEST DNA polymerase (Takara Shuzo) was prepared. The reaction mixture was incubated at 58°C for 1 hour in a thermal cycler.

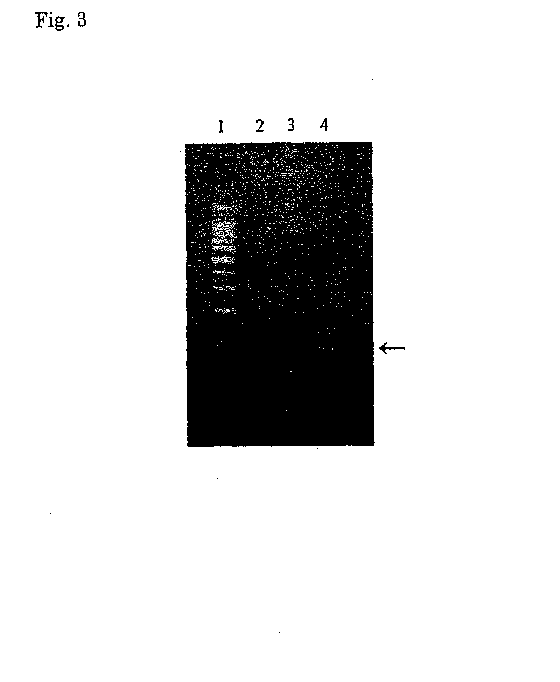

5 µl each of the reaction mixtures was analyzed by electrophoresis on 3% agarose gel. The results are shown in Figure 3.

Figure 3 is a photograph of the electrophoresis of the reaction products. Lane 1: 100 bp DNA ladder marker; lane 2: negative control (water); lane 3: 0.115 ng of the template; and lane 4: 1.15 ng of the template. It was confirmed that a specific amplification product was generated when 1.15 ng of the template DNA was used in the reaction. Thus, it was shown that it is possible to create a replication origin on a genomic DNA according to the method of the present invention.

### Example 4

(1) An ecotropic retrovirus was prepared from a culture supernatant of a packaging cell GP+E-86 (ATCC CRL-9642) into which a plasmid pDON-AI had been introduced by a calcium phosphate method. NIH/3T3 cell (ATCC CRL-1658) was infected with the retrovirus and cultured in a medium containing G418 for 14 days to prepare a transformed cell.. 27 µg of a genomic DNA was prepared from 4 x 10⁴ of the retrovirus-infected cells according to a conventional method.
(2) Preparation of primers
   Two chimeric oligonucleotide primers pDON-AI-68-1 (sense primer) and pDON-AI-68-2 (antisense primer) each having three RNA bases at the 3' terminus were synthesized based on the nucleotide sequence of the packaging region in a plasmid pDON-AI. The nucleotide sequences of pDON-AI-68-1 and pDON-AI-68-2 are shown in SEQ ID NOS:14 and 15, respectively.
(3) Amplification of DNA fragment without denaturation of template
   50 µl of a reaction mixture containing 1 µl of a solution containing 10 fg or 1 ng of pDON-AI, 1 µl of a solution containing 1 ng, 10 ng or 100 ng of the genomic DNA from NIH/3T3 cells having pDON-AI being introduced, or 1 µl of water for a negative control, 50 pmol each of the primers prepared in Example 4-(2) above, 0.5 mM each of dNTPs, 32 mM HEPES-potassium hydroxide buffer (pH 7.8), 100 mM potassium acetate, 4 mM magnesium acetate, 0.01% bovine serum albumin, 1% dimethyl sulfoxide, 18.5 U of Pfu RNase HII as described in Referential Example and 4 U of BcaBEST DNA polymerase (Takara Shuzo) was prepared. The reaction mixture was incubated at 64°C for 1 hour in a thermal cycler.

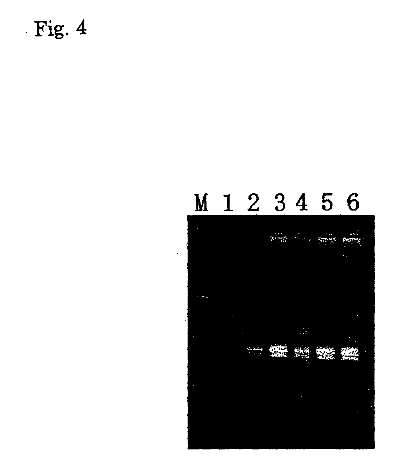

After reaction, 5 µl each of the reaction mixtures was subjected to electrophoresis on 3% agarose gel to confirm the reaction product. The results are shown in Figure 4.

Figure 4 is a photograph of the electrophoresis of the reaction products. Lane M: 100 bp DNA ladder marker; lane 1: negative control (water); lane 2: 10 fg of pDON-AI as a template; lane 3: 1 pg of pDON-AI as a template; lane 4: 1 ng of the genomic DNA from NIH/3T3 cells having pDON-AI as a template; lane 5: 10 ng of the genomic DNA from NIH/3T3 cells having pDON-AI as a template; and lane 6: 100 ng of the genomic DNA from NIH/3T3 cells having pDON-AI as a template.

As shown in Figure 4, specific amplification of a DNA fragment was observed using either pDON-AI or the genomic DNA containing pDON-AI being integrated. Thus, it was shown that it is possible to amplify a DNA fragment of interest without denaturing a template DNA prior to the reaction even if a genomic DNA is used as a template.

### Example 5

### (1) Synthesis of chimeric oligonucleotide and primer

A chimeric oligonucleotide k-ras-X having three RNA residues at the 3' terminus and labeled at the 5' terminus with X-rhodamine isothiocyanate (XRITC) was designed and synthesized based on the nucleotide sequence of human c-Ki-ras gene. The nucleotide sequences of k-ras-X is shown in SEQ ID NO:16.

Primers ras-F (sense) and ras-R (antisense) were synthesized as a pair of primers for a PCR for preparing a PCR fragment to be used as a template. The nucleotide sequences of ras-F and ras-R are shown in SEQ ID NOS:17 and 18, respectively.

### (2) Reaction of chimeric oligonucleotide with template

A PCR was carried out using 1 µl of a human genomic DNA as a template and the primers ras-F and ras-R to synthesize a DNA fragment to be used as a template which had a sequence complementary to k-ras-X. 50 µl of a reaction mixture with the following composition containing 270 ng of this DNA fragment and 25 pmol of k-ras-X was prepared: 0.5 mM each of dNTPs, 32 mM HEPES-potassium hydroxide buffer (pH 7.8), 100 mM potassium acetate, 4 mM magnesium acetate, 0.01% bovine serum albumin and 1% dimethyl sulfoxide. The reaction mixture was incubated at 52°C for 5 minutes in a thermal cycler. A reaction mixture to which a 200-bp PCR-amplified fragment without a sequence complementary to k-ras-X was added was prepared as a negative control, and was incubated in a similar manner.

20 µl of the reaction mixture was subjected to electrophoresis on 5% nondenaturing polyacrylamide gel. After electrophoresis, the gel was analyzed using FMBIO-II Multi-View (Takara Shuzo) to confirm the electrophoretic position of k-ras-X. As a result, in case of incubation with the DNA fragment having the complementary nucleotide sequence, fluorescence from k-ras-X was observed at an electrophoretic position corresponding to the size of the DNA fragment, confirming that the chimeric oligonucleotide formed a complex with the DNA fragment. On the other hand, no such binding of the primer to the DNA fragment was observed for the negative control. Thus, it was shown that a chimeric oligonucleotide having a nucleotide sequence complementary to a DNA fragment specifically binds to the nucleotide sequence of the DNA fragment without denaturing the DNA fragment in an enzyme-independent manner.

### Industrial Applicability

The present invention provides a method for artificially creating a replication origin on a nucleic acid molecule and replicating the nucleic acid molecule utilizing the replication origin.

According to the method of the present invention, a nucleic acid molecule can be conveniently replicated in vitro without inserting it into a vector. Furthermore, since a replication origin can be positioned at will, it is possible to specifically replicate only a necessary portion of a nucleic acid, or to conduct replication reactions from plurality of replication origins at the same time.

### Sequence Listing Free Text

SEQ ID NO:2: PCR primer 1650Nde for cloning a gene encoding a polypeptide having a RNase HII activity from Pyrococcus furiosus
SEQ ID NO:3: PCR primer 1650Bam for cloning a gene encoding a polypeptide having a RNase HII activity from Pyrococcus furiosus
SEQ ID NO:6: Designed oligonucleotide primer to amplify a portion of iNOS-encoding sequence from mouse
SEQ ID NO:7: Designed oligonucleotide primer to amplify a portion of iNOS-encoding sequence from mouse
SEQ ID NO:8: Designed chimeric oligonucleotide primer to amplify a portion of iNOS-encoding sequence from mouse. "nucleotides 21 to 23 are ribonucleotides-other nucleotides are deoxyribonucleotides"
SEQ ID NO:9: Designed chimeric oligonucleotide primer to amplify a portion of iNOS-encoding sequence from mouse. "nucleotides 19 to 22 are ribonucleotides-other nucleotides are deoxyribonucleotides"
SEQ ID NO:10: Designed chimeric oligonucleotide primer to amplify a portion of plasmid pDON-AI. "nucleotides 17 to 19 are ribonucleotides-other nucleotides are deoxyribonucleotides"
SEQ ID NO:11: Designed chimeric oligonucleotide primer to amplify a portion of plasmid pDON-AI. "nucleotides 19 to 21 are ribonucleotides-other nucleotides are deoxyribonucleotides"
SEQ ID NO:12: Designed chimeric oligonucleotide primer to amplify a portion of Genomic DNA of Staphylococcus aureus. "nucleotides 19 to 21 are ribonucleotides-other nucleotides are deoxyribonucleotides"
SEQ ID NO:13: Designed chimeric oligonucleotide primer to amplify a portion of Genomic DNA of Staphylococcus aureus. "nucleotides 19 to 21 are ribonucleotides-other nucleotides are deoxyribonucleotides"

## Claims

1. A method for forming a complex consisting of a double-stranded nucleic acid and an oligonucleotide, the method comprising:
(a) mixing a double-stranded nucleic acid and at least one oligonucleotide to prepare a reaction mixture, wherein the oligonucleotide is a chimeric oligonucleotide containing at least a ribonucleotide as well as a member selected from a group consisting of a deoxyribonucleotide and a nucleotide analog, and has a sequence substantially complementary to the nucleotide sequence of one of the two strands of the double-stranded nucleic acid; and
(b) incubating the reaction mixture to form a complex under conditions under which the double-stranded nucleic acid is not denatured.

2. The method according to claim 1, wherein the double-stranded nucleic acid is a nucleic acid selected from the group consisting of a linear DNA, a circular DNA and a genomic DNA.

3. The method according to claim 1, wherein the oligonucleotide can function as a primer for synthesizing a DNA complementary to one of the two strands of the double-stranded nucleic acid.

4. The method according to claim 1, wherein the oligonucleotide is labeled.

5. A method for detecting a double-stranded nucleic acid having a target nucleotide sequence, the method comprising:
(a) forming a complex consisting of a double-stranded nucleic acid and an oligonucleotide according to the method defined by claim 1, wherein the oligonucleotide is a chimeric oligonucleotide containing at least a ribonucleotide as well as a member selected from a group consisting of a deoxyribonucleotide and a nucleotide analog, and has a sequence substantially complementary to a target nucleotide sequence; and
(b) detecting the oligonucleotide forming the complex.

6. A method for creating a replication origin on a double-stranded nucleic acid, the method comprising:
(a) mixing a double-stranded nucleic acid and at least one oligonucleotide to prepare a reaction mixture, wherein the oligonucleotide is a chimeric oligonucleotide containing at least a ribonucleotide as well as a member selected from a group consisting of a deoxyribonucleotide and a nucleotide analog, allows extension from its 3' terminus by a DNA polymerase, and has a sequence substantially complementary to the nucleotide sequence of one of the two strands of the double-stranded nucleic acid; and
(b) incubating the reaction mixture to form a complex under conditions under which the double-stranded nucleic acid is not denatured.

7. The method according to claim 6, wherein the double-stranded nucleic acid is a nucleic acid selected from the group consisting of a linear DNA, a circular DNA and a genomic DNA.

8. A method for replicating a nucleic acid, the method comprising:
synthesizing a DNA complementary to one of the two strands of a double-stranded nucleic acid from a replication origin created according to the method defined by claim 6 in the presence of a DNA polymerase.

9. The method according to claim 8, wherein the DNA polymerase is an enzyme that has a strand displacement activity.


SEQUENCE LISTING

<110> Takara Bio Inc.

<120> Method of Forming complex

<130> 38-57

<150> JP 2000-284420
<151> 2000-09-19

<150> JP 2001-139286
<151> 2001-05-09

<150> JP 2001-177537
<151> 2001-06-12

<160> 18

<210> 1
<211> 663
<212> DNA
<213> Pyrococcus horikoshii

<400> 1
atgaaggttg ctggagttga tgaagcgggg agggggccgg taattggccc gttagtaatt 60
ggagtagccg ttatagatga gaaaaatatt gagaggttac gtgacattgg ggttaaagac 120
tccaaacaat taactcctgg gcaacgtgaa aaactattta gcaaattaat agatatccta 180
gacgattatt atgttcttct cgttaccccc aaggaaatag atgagaggca tcattctatg 240

aatgaactag aagctgagaa attcgttgta gccttgaatt ctttaaggat caagccgcag 300
aagatatatg tggactctgc cgatgtagat cctaagaggt ttgctagtct aataaaggct 360
gggttgaaat atgaagccac ggttatcgcc gagcataaag ccgatgcaaa gtatgagata 420
gtatcggcag catcaataat tgcaaaggtc actagggata gagagataga gaagctaaag 480
caaaagtatg gggaatttgg ttctggctat ccgagtgatc cgagaactaa ggagtggctt 540
gaagaatatt acaaacaata tggtgacttt cctccaatag ttaggagaac ttgggaaacc 600
gctaggaaga tagaggaaag gtttagaaaa aatcagctaa cgcttgataa attccttaag 660
tga 663

<210> 2
<211> 33
<212> DNA
<213> Artificial Sequence

<220>
<223> PCR primer 1650Nde for cloning a gene encoding a polypeptide having a
RNaseHII activity from Pyrococcus furiosus

<400> 2
caggaggaga gacatatgaa aataggggga att 33

<210> 3
<211> 33
<212> DNA
<213> Artificial Sequence

<220>
<223> PCR primer 1650Bam for cloning a gene encoding a polypeptide having a
RNaseHII activity from Pyrococcus furiosus

<400> 3

gaaggttgtg gatccacttt ctaaggtttc tta     33

<210> 4

<211> 672

<212> DNA

<213> Pyrococcus furiosus

<400> 4

atgaaaatag ggggaattga cgaagcagga agaggaccag cgatagggcc attagtagta     60

gctactgtcg tcgttgatga gaaaaacatt gagaagctca gaaacattgg agtaaaagac     120

tccaaacaac taacacccca tgaaaggaag aatttatttt cccagataac ctcaatagcg     180

gatgattaca aaatagtgat agtatcccca gaagaaatcg acaatagatc aggaacaatg     240

aacgagttag aggtagagaa gtttgctctc gccttaaatt cgcttcagat aaaaccagct     300

cttatatacg ctgatgcagc ggatgtagat gccaatagat ttgcaagctt gatagagaga     360

agactcaatt ataaggcgaa gattattgcc gaacacaagg ccgatgcaaa gtatccagta     420

gtttcagcag cttcaatact tgcaaaggtt gttagggatg aggaaattga aaaattaaaa     480

aagcaatatg gagactttgg ctctgggtat ccaagtgatc caaaaaccaa gaaatggctt     540

gaagagtact acaaaaaaca caactctttc cctccaatag tcagacgaac ctgggaaact     600

gtaagaaaaa tagaggaaag cattaaagcc aaaaaatccc agctaacgct tgataaattc     660

tttaagaaac ct                                                        672

<210> 5

<211> 224

<212> PRT

<213> Pyrococcus furiosus

<400> 5

Met Lys Ile Gly Gly Ile Asp Glu Ala Gly Arg Gly Pro Ala Ile
1               5                   10                  15
Gly Pro Leu Val Val Ala Thr Val Val Val Asp Glu Lys Asn Ile
                20                  25                  30
Glu Lys Leu Arg Asn Ile Gly Val Lys Asp Ser Lys Gln Leu Thr
                35                  40                  45
Pro His Glu Arg Lys Asn Leu Phe Ser Gln Ile Thr Ser Ile Ala
                50                  55                  60
Asp Asp Tyr Lys Ile Val Ile Val Ser Pro Glu Glu Ile Asp Asn
                65                  70                  75
Arg Ser Gly Thr Met Asn Glu Leu Glu Val Glu Lys Phe Ala Leu
                80                  85                  90
Ala Leu Asn Ser Leu Gln Ile Lys Pro Ala Leu Ile Tyr Ala Asp
                95                  100                 105
Ala Ala Asp Val Asp Ala Asn Arg Phe Ala Ser Leu Ile Glu Arg
                110                 115                 120
Arg Leu Asn Tyr Lys Ala Lys Ile Ile Ala Glu His Lys Ala Asp
                125                 130                 135
Ala Lys Tyr Pro Val Val Ser Ala Ala Ser Ile Leu Ala Lys Val
                140                 145                 150
Val Arg Asp Glu Glu Ile Glu Lys Leu Lys Lys Gln Tyr Gly Asp
                155                 160                 165
Phe Gly Ser Gly Tyr Pro Ser Asp Pro Lys Thr Lys Lys Trp Leu
                170                 175                 180
Glu Glu Tyr Tyr Lys Lys His Asn Ser Phe Pro Pro Ile Val Arg
                185                 190                 195
Arg Thr Trp Glu Thr Val Arg Lys Ile Glu Glu Ser Ile Lys Ala
                200                 205                 210
Lys Lys Ser Gln Leu Thr Leu Asp Lys Phe Phe Lys Lys Pro

                215                 220

<210> 6

<211> 21

<212> DNA

<213> Artificial Sequence

<220>

<223> Designed oligonucleotide primer to amplify a portion of iNOS-encoding

sequence from mouse

<400> 6

cacaaggcca catcggattt c 21

<210> 7

<211> 21

<212> DNA

<213> Artificial Sequence

<220>

<223> Designed oligonucleotide primer to amplify a portion of iNOS-encoding

sequence from mouse

<400> 7

tgcataccac ttcaacccga g 21

<210> 8

<211> 23

<212> DNA

<213> Artificial Sequence

<220>
<223> Designed chmeric oligonucleotide primer to amplify a portion of iNOS-encoding
sequence from mouse.   “nucleotides 21 to 23 are ribonucleotides-other nucleotides are
deoxyribonucleotides”

<400> 8
ctcatgccat tgagttcatc aac          23

<210> 9
<211> 22
<212> DNA
<213> Artificial Sequence

<220>
<223> Designed chmeric oligonucleotide primer to amplify a portion of iNOS-encoding
sequence from mouse.   “nucleotides 19 to 22 are ribonucleotides-other nucleotides are
deoxyribonucleotides”

<400> 9
gctggtaggt tcctgttgtu uc          22

<210> 10
<211> 19
<212> DNA
<213> Artificial Sequence

<220>

<223> Designed chmeric oligonucleotide primer to amplify a portion of plasmid pDON-
AI. “nucleotides 17 to 19 are ribonucleotides-other nucleotides are
deoxyribonucleotides”

<400> 10
agctctgtat ctggcggac 19

<210> 11
<211> 21
<212> DNA
<213> Artificial Sequence

<220>
<223> Designed chmeric oligonucleotide primer to amplify a portion of plasmid pDON-
AI. “nucleotides 19 to 21 are ribonucleotides-other nucleotides are
deoxyribonucleotides”

<400> 11
gatcgggatt tttggactca g 21

<210> 12
<211> 21
<212> DNA
<213> Artificial Sequence

<220>
<223> Designed chmeric oligonucleotide primer to amplify a portion of Genomic DNA
of Staphylococcus aureus. “nucleotides 19 to 21 are ribonucleotides-other nucleotides
are deoxyribonucleotides”

<400> 12

tgtatgtatg gtggtgtaac g          21

<210> 13

<211> 21

<212> DNA

<213> Artificial Sequence

<220>

<223> Designed chmeric oligonucleotide primer to amplify a portion of Genomic DNA

of Staphylococcus aureus.   "nucleotides 19 to 21 are ribonucleotides-other nucleotides

are deoxyribonucleotides"

<400> 13

taaccgtttc caaaggtacu g          21

<210> 14

<211> 22

<212> DNA

<213> Artificial Sequence

<220>

<223> Designed chmeric oligonucleotide primer to amplify a portion of plasmid pDON-

AI.   "nucleotides 20 to 22 are ribonucleotides-other nucleotides are

deoxyribonucleotides"

<400> 14

actagctctg tatctggcgg ac          22

<210> 15

<211> 23

<212> DNA

<213> Artificial Sequence

<220>

<223> Designed chmeric oligonucleotide primer to amplify a portion of plasmid pDON-

AI. "nucleotides 21 to 23 are ribonucleotides-other nucleotides are

deoxyribonucleotides"

<400> 15

acgatcggga tttttgcact cag 23

<210> 16

<211> 20

<212> DNA

<213> Artificial Sequence

<220>

<223> Designed chmeric oligonucleotide complementary to human ki-ras gene.

"nucleotides 18 to 20 are ribonucleotides-other nucleotides are deoxyribonucleotides"

<400> 16

gactgaatat aaacttgugg 20

<210> 17

<211> 22

<212> DNA

<213> Artificial Sequence

<220>
<223> Designed oligonucleotide primer to amplify a portion of human ki-ras gene.

<400> 17
tgacatgttc taatatagtc ac                    22

<210> 18
<211> 20
<212> DNA
<213> Artificial Sequence

<220>
<223> Designed oligonucleotide primer to amplify a portion of human ki-ras gene.

<400> 18
actcatgaaa atggtcagag                    20